# EUROPEAN PATENT APPLICATION

(11) **EP 4 272 856 A1**
(43) Date of publication of application: **08.11.2023**
(21) Application number: 22749650.2
(22) Date of filing: 31.01.2022
(51) Int. Cl.: B01D 63/00, B01D 63/02, A61M 1/18

(54) **ARTIFICIAL LUNG AND METHOD FOR MANUFACTURING ARTIFICIAL LUNG**

(30) Priority: 05.02.2021 JP 2021017499
(71) Applicant: TERUMO Kabushiki Kaisha, Tokyo 151-0072 (JP)
(72) Inventor: IWASE, Yoji, Fujinomiya-shi, Shizuoka 418-0015 (JP)
(74) Representative: Casalonga
(86) International application number: PCT/JP2022/003554
(87) International publication number: WO 2022/168778

(57) **Abstract**

An oxygenator (10) includes: a hollow fiber module (19) having a wound hollow fiber membrane; a cylindrical outer tube (22) that accommodates the hollow fiber module (19); and a sealing structure (82a, 82b) that seals a gap between an outer peripheral portion of the hollow fiber module (19) and an inner peripheral portion of the outer tube (22), the sealing structure (82a, 82b) including an anchor structure (84a, 84b) that is formed on the outer tube (22) on an inner peripheral side near the end and that has a groove recessed in an axial direction of the outer tube (22), a cutout portion (88) formed by cutting out the anchor structure (84a, 84b) on the inner peripheral side, and a sealing material (86a, 86b) with which the anchor structure (84a, 84b) is filled.

## Description

### Technical Field

The present invention relates to an oxygenator for removing carbon dioxide in blood through a hollow fiber membrane and adding oxygen to the blood, and a method for manufacturing the oxygenator.

### Background Art

A hollow fiber membrane oxygenator using a porous membrane is generally and widely used as an extracorporeal circulation device or an artificial heart-lung machine for assisting circulation in open-heart surgery or the like of heart disease. The membrane oxygenator mainly uses a hollow fiber membrane, and performs gas exchange and heat exchange of blood through the hollow fiber membrane.

In the oxygenator, the hollow fiber membrane is wound around a cylindrical member and accommodated in a cylindrical outer tube. Both ends of the outer tube are filled with a potting material such as a urethane resin in order to seal an internal space that accommodates the hollow fiber membrane (for example, JP 2002-35116 A).

### Summary of Invention

Regarding the potting material, a liquid potting material (hereinafter also referred to as a sealing material) is poured into a gap between the outer tube and the hollow fiber membrane under the centrifugal action. Thereafter, the liquid sealing material is cured to form a sealing structure. Since the sealing material contracts during a manufacturing process, a groove-shaped anchor structure having protrusions and recesses is provided inside the outer tube in order to prevent separation between the outer tube and the sealing material.

However, bubbles are not sufficiently removed due to the anchor structure, and large bubbles may remain in the sealing material near the anchor structure. Such bubbles in the sealing material do not affect sealing performance, but are visible in appearance, and thus may give a user a sense of anxiety.

In view of this, an object of the present invention is to provide an oxygenator capable of preventing bubbles from remaining in a sealing material and a method for manufacturing the oxygenator.

One aspect of the present invention provides an oxygenator including: a hollow fiber module having a plurality of hollow fiber membranes; an outer tube that is cylindrical and that accommodates the hollow fiber module; and a sealing structure that is provided at an end of the outer tube and seals a gap between an outer peripheral portion of the hollow fiber module and an inner peripheral portion of the outer tube, wherein the sealing structure includes an anchor structure that is formed on the outer tube on an inner peripheral side near the end and that has a groove recessed in an axial direction of the outer tube, a cutout portion formed by cutting out the groove of the anchor structure on the inner peripheral side, and a sealing material with which the anchor structure and the gap are filled.

Another aspect provides a method for manufacturing an oxygenator that includes a hollow fiber module having a plurality of hollow fiber membranes, an outer tube that is cylindrical and that accommodates the hollow fiber module, and a sealing structure that is provided at an end of the outer tube and that seals a gap between an outer peripheral portion of the hollow fiber module and an inner peripheral portion of the outer tube, the sealing structure including an anchor structure that is formed on the outer tube on an inner peripheral side near the end and that has a groove recessed in an axial direction of the outer tube, a cutout portion formed by cutting out the groove of the anchor structure on the inner peripheral side, and a sealing material with which the anchor structure and the gap are filled, the method including: accommodating the hollow fiber module into the outer tube; and filling the groove and the gap between the outer tube and the hollow fiber module with the sealing material while applying a centrifugal force in the axial direction of the outer tube.

The oxygenator and the method for manufacturing the oxygenator according to the above aspects can prevent bubbles from remaining in the sealing material.

### Brief Description of Drawings

Fig. 1 is a longitudinal sectional view of an oxygenator according to a first embodiment.
Fig. 2 is an enlarged cross-sectional view of the vicinity of the outer periphery of the oxygenator illustrated in Fig. 1.
Fig. 3 is a perspective view of an outer tube in Fig. 1.
Fig. 4 is a front view of the outer tube in Fig. 1.
Fig. 5 is an enlarged cross-sectional view of an anchor structure in Fig. 4.
Fig. 6A is an enlarged perspective view illustrating a cutout portion of the anchor structure, and Fig. 6B is a sectional perspective view of the cutout portion.
Fig. 7 is a cross-sectional view illustrating a step for accommodating a hollow fiber module in the outer tube in a method for manufacturing the oxygenator illustrated in Fig. 1.
Fig. 8 is a cross-sectional view illustrating a step for filling a gap between the outer tube and the hollow fiber module with a potting material in the method for manufacturing the oxygenator illustrated in Fig. 1.
Fig. 9 is an explanatory diagram illustrating a direction in which a centrifugal force is applied in the step for filling the gap with the potting material.
Fig. 10 is an explanatory diagram illustrating a distance relationship between the anchor structure of the outer tube and a rotation center.
Figs. 11A to 11C are explanatory diagrams illustrating the movement of the potting material and bubbles at the end of the outer tube in the order of the steps.
Fig. 12 is a photograph of the vicinity of an anchor structure of an oxygenator according to Examination Example 1 (Comparative Example).
Fig. 13 is a photograph of the vicinity of an anchor structure of an oxygenator according to Examination Example 2 (first embodiment).
Fig. 14 is a schematic diagram of an outer tube according to a second embodiment.

### Description of Embodiments

Preferred embodiments of an oxygenator and a method for manufacturing the oxygenator will be described below in detail with reference to the accompanying drawings.

### (First Embodiment)

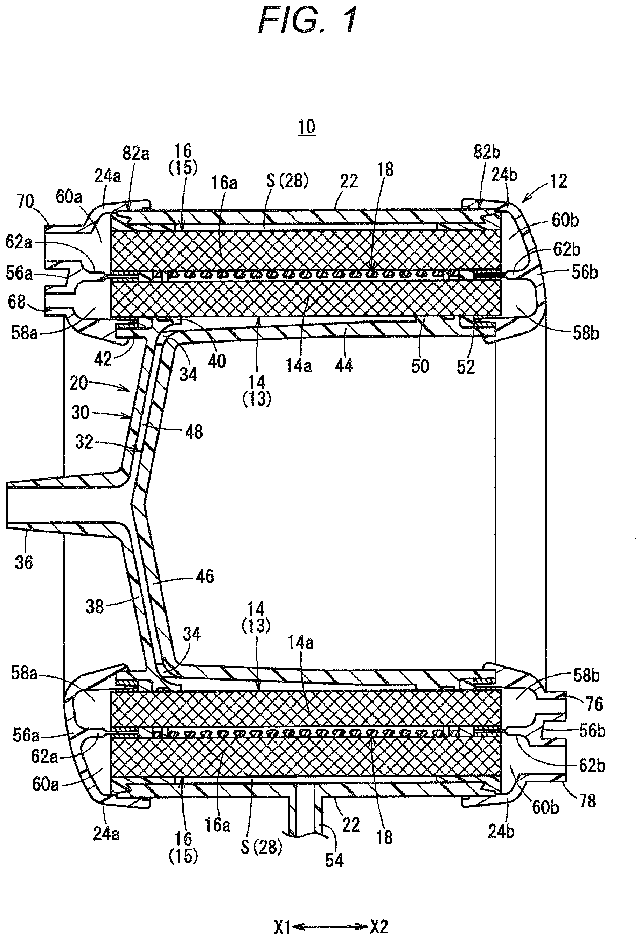

As illustrated in Fig. 1, an oxygenator 10 according to the present embodiment is a medical device that temporarily functions as a lung during an operation such as cardiac surgery of a human body. Specifically, the oxygenator 10 is a device for adjusting a blood temperature, removing carbon dioxide in the blood, and supplying oxygen into the blood in extracorporeal blood circulation.

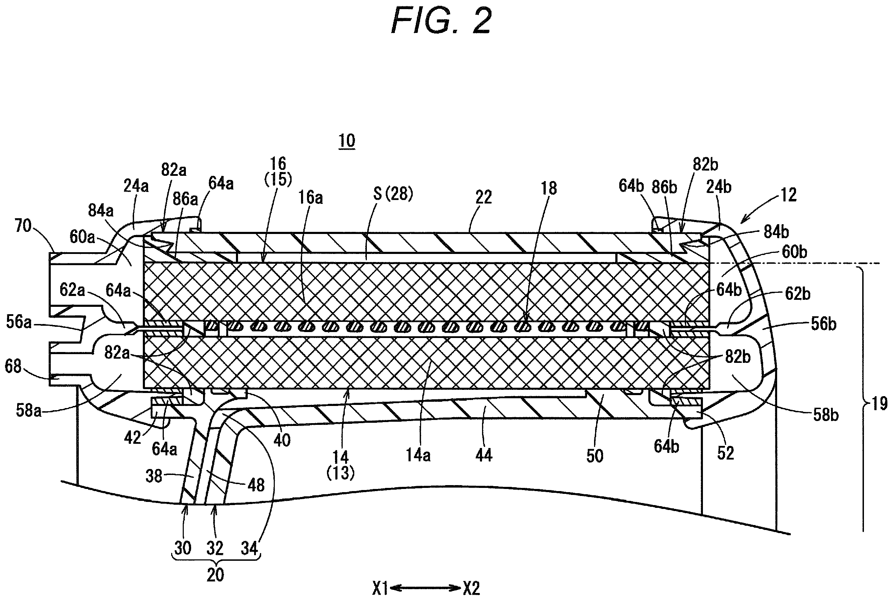

As illustrated in Figs. 1 and 2, the oxygenator 10 includes a housing 12, a heat exchanger 14 which is an inner tube portion 13, a gas exchanger 16 which is an outer tube portion 15, and an intermediate spacer 18. The heat exchanger 14 and the gas exchanger 16 include a plurality of hollow fiber membranes and are joined with the intermediate spacer 18 therebetween. The heat exchanger 14, the intermediate spacer 18, and the gas exchanger 16 which are wound around a core 20 constitute a hollow fiber module 19.

In Fig. 1, the housing 12 includes the core 20 constituting a central portion of the oxygenator 10, an outer tube 22 provided on an outer peripheral side of the core 20, a first cover member 24a attached to one end of the core 20 and one end of the outer tube 22, and a second cover member 24b attached to the other end of the core 20 and the other end of the outer tube 22.

The core 20, the outer tube 22, the first cover member 24a, and the second cover member 24b form an accommodation space S for accommodating the cylindrical heat exchanger 14 and the cylindrical gas exchanger 16. The accommodation space S functions as a blood flow path 28. Each of the core 20, the outer tube 22, the first cover member 24a, and the second cover member 24b is formed of a hard resin.

The core 20 includes a first core portion 30 constituting one end of the core 20 and a second core portion 32 constituting the other end of the core 20. The first core portion 30 and the second core portion 32 are coupled to each other by a plurality of connecting portions 34.

The first core portion 30 has a blood inflow portion 36 to which a tube (not illustrated) can be connected at one end, and an annular wall 38 extending radially outward from the blood inflow portion 36. The outer end of the wall 38 is provided with a first support portion 40 for supporting the heat exchanger 14, and a first annular protrusion 42 protruding from the first support portion 40 to one side of the outer tube 22 in the axial direction (direction of arrow X1 in Fig. 1).

The second core portion 32 is formed in a bottomed cylindrical shape, and includes a cylindrical portion 44 and a closing portion 46 provided at one end (end in the direction of arrow X1) of the cylindrical portion 44. The closing portion 46 is disposed so as to face the wall 38 with a gap therebetween. The gap between the closing portion 46 and the wall 38 functions as a blood introduction path 48 for guiding the blood flowing in through the blood inflow portion 36 into the accommodation space S. The outer surface of the cylindrical portion 44 is provided with a second support portion 50 for supporting the heat exchanger 14, and a second annular protrusion 52 protruding from the second support portion 50 to the other side of the outer tube 22 in the axial direction (direction of arrow X2 in Fig. 1). The second support portion 50 is located at the other end of the cylindrical portion 44.

As illustrated in Fig. 2, a first partition wall 62a annularly extends and protrudes in the axial direction from the inner surface of a first cover main body 56a toward the side where the heat exchanger 14 and the gas exchanger 16 are located. The protruding end of the first partition wall 62a is fixed by an adhesive 64a in a state of being inserted between one end of the heat exchanger 14 and one end of the gas exchanger 16.

As illustrated in Fig. 2, a first sealing structure 82a for sealing blood inside the accommodation space S is provided on one end side of the outer tube 22. The first sealing structure 82a prevents leakage of blood in the accommodation space S into a first gas flow path 60a. In addition, a second sealing structure 82b for sealing blood inside the accommodation space S is provided on the other end side of the outer tube 22. The second sealing structure 82b prevents leakage of blood in the accommodation space S into a second gas flow path 60b.

The first sealing structure 82a includes a first anchor structure 84a having a groove-shaped recess provided on the inner peripheral side of the outer tube 22, and a first sealing material 86a with which a gap between the outer tube 22 and the gas exchanger 16 is filled. In addition, the second sealing structure 82b includes a second anchor structure 84b having a groove-shaped recess provided on the inner peripheral side of the outer tube 22, and a second sealing material 86b with which a gap between the outer tube 22 and the gas exchanger 16 is filled.

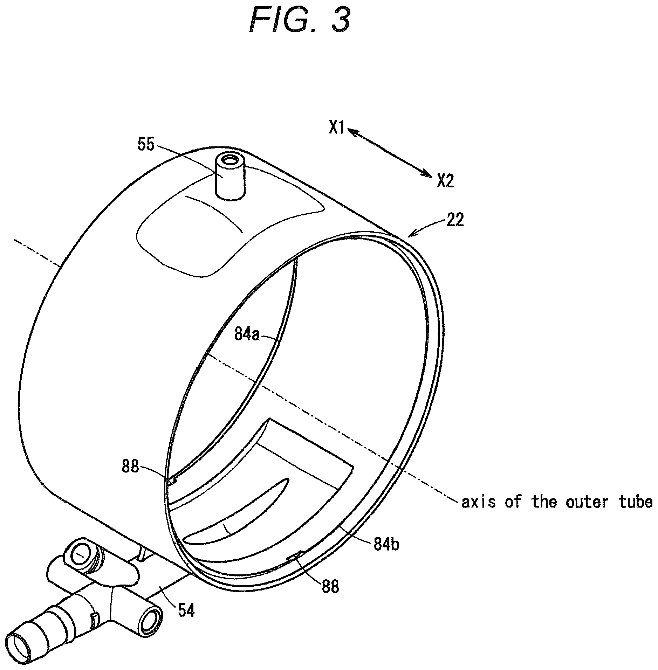

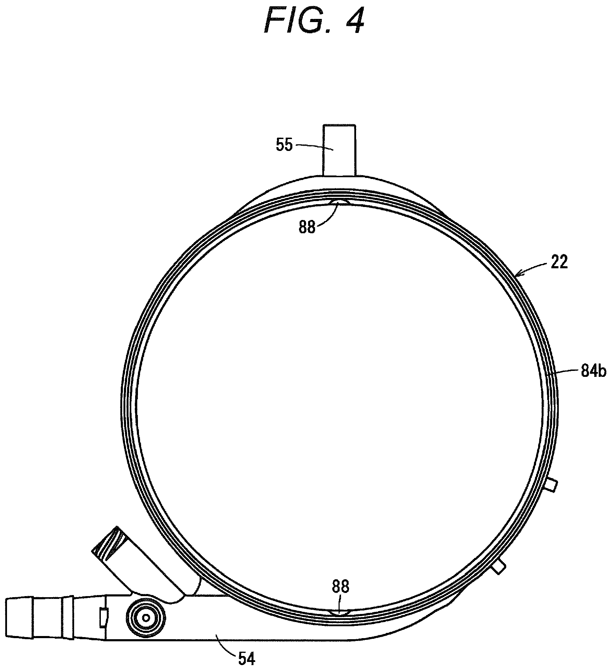

As illustrated in Fig. 3, the second anchor structure 84b of the second sealing structure 82b includes a groove formed in the outer tube 22 on the inner peripheral side in the vicinity of the other end. As illustrated in Fig. 4, the second anchor structure 84b is formed over the entire circumferential area of the inner periphery of the cylindrical outer tube 22, and is formed in a circular shape when viewed in the axial direction. The groove constituting the second anchor structure 84b is constant in position of the outer tube 22 in the axial direction, and is separated from the other end of the outer tube 22 by a constant distance in the axial direction.

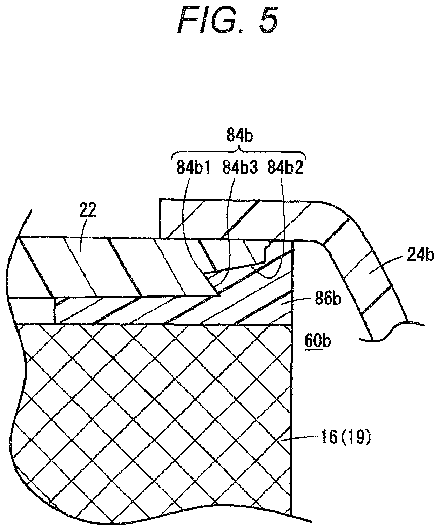

As illustrated in Fig. 5, the second anchor structure 84b has a bottom portion 84b1 constituting the bottom of the groove. An outer inclined surface 84b2 inclined so as to gradually increase in diameter as it extends to the end of the outer tube 22 is formed on the outer peripheral side of the bottom portion 84b1. An inner inclined surface 84b3 meeting the outer inclined surface 84b2 at an acute angle is formed on the inner peripheral side of the bottom portion 84b1. The bottom portion 84b1 is formed as a ridge portion where the outer inclined surface 84b2 and the inner inclined surface 84b3 meet. The second anchor structure 84b prevents separation of the second sealing material 86b due to thermal contraction.

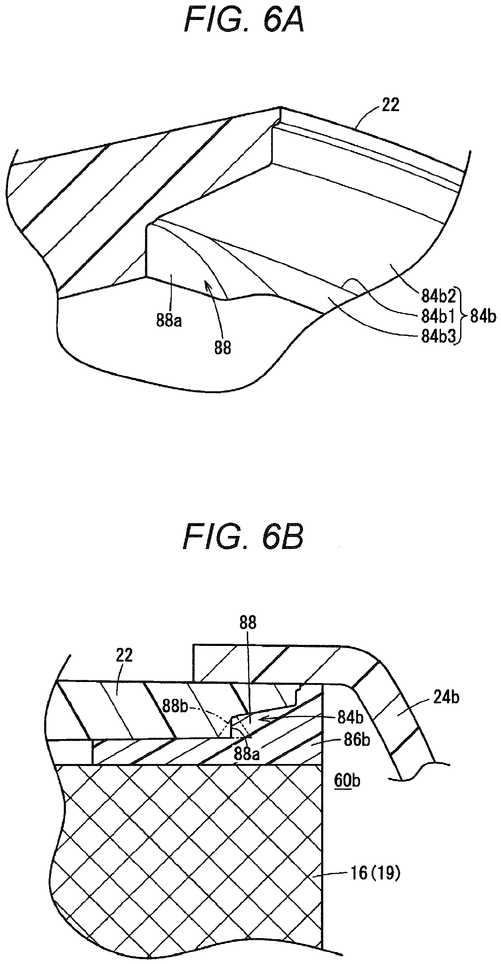

As illustrated in Fig. 4, a cutout portion 88 formed by cutting out the groove constituting the second anchor structure 84b is formed in a part of the second anchor structure 84b in the circumferential direction. As illustrated in Fig. 6A, the cutout portion 88 is formed by cutting out a portion of a protrusion constituting the inner inclined surface 84b3 constituting the second anchor structure 84b. As illustrated in Fig. 6B, a bottom surface 88a of the cutout portion 88 is defined by a flat surface perpendicular to the axial direction of the outer tube 22. The bottom surface 88a is provided on the inner side in the axial direction than the bottom portion 84b1 of the second anchor structure 84b, and when a centrifugal force toward the inner side in the axial direction is applied, a potting material (sealing material 86) introduced into the second anchor structure 84b can be discharged.

Note that the bottom surface 88a is not limited to be perpendicular to the axial direction of the outer tube 22, and may be inclined with respect to the axial direction of the outer tube 22. In this case, the bottom surface 88a on the inner peripheral side may be inclined toward the inner side of the outer tube 22 as indicated by an imaginary line 88b in Fig. 6B from the viewpoint of the discharging efficiency of the sealing material 86 (potting material).

As illustrated in Fig. 4, a pair of cutout portions 88 is provided at positions facing each other across the central axis of the outer tube 22. Here, when the oxygenator is placed such that a blood outflow port 54 of the outer tube 22 is located at a lower end and a prime auxiliary port 55 is located at an upper end, the cutout portions are provided at the 0 o'clock position (upper end position) and the 6 o'clock position (lower end position). Note that the number of cutout portions 88 to be formed is not limited to one pair (two), and may be three or more. When a plurality of pairs of cutout portions 88 is provided, the paired cutout portions 88 are preferably disposed at positions facing each other across the central axis of the outer tube 22.

As illustrated in Figs. 5 and 6B, a gap between the outer tube 22 and the other end of the gas exchanger 16 is filled with the second sealing material 86b. The entire region of the second anchor structure 84b including the cutout portions 88 is filled with the second sealing material 86b. The other end of the second sealing material 86b has substantially the same height as the other end of the gas exchanger 16. As the second sealing material 86b, a urethane resin or the like can be used, for example.

As illustrated in Fig. 3, the first anchor structure 84a is formed on one end side of the outer tube 22. The first anchor structure 84a is configured similarly to the second anchor structure 84b, and has the cutout portions 88 at the same positions in the circumferential direction. As illustrated in Fig. 2, the first anchor structure 84a is filled with the first sealing material 86a. The first sealing material 86a is configured similarly to the second sealing material 86b. In the present specification, the first sealing material 86a and the second sealing material 86b are collectively referred to as a sealing material 86.

Next, the operation of the oxygenator 10 configured as described above will be described.

As illustrated in Fig. 2, a heat medium is supplied to a heat medium inflow portion 68 of the oxygenator 10, oxygen is supplied to a gas inflow portion 70, and blood from a human body is introduced into the blood inflow portion 36 through a centrifugal pump or a roller pump (not illustrated).

The heat medium supplied from the heat medium inflow portion 68 is introduced into a lumen of a first hollow fiber membrane 14a of the heat exchanger 14 through a first heat medium flow path 58a. The heat medium exchanges heat with blood when passing through the lumen of the first hollow fiber membrane 14a. The heat medium having passed through the first hollow fiber membrane 14a is collected in a second heat medium flow path 58b and flows out of the oxygenator 10 through a heat medium outflow portion 76.

Oxygen supplied from the gas inflow portion 70 is introduced into the lumen of a second hollow fiber membrane 16a of the gas exchanger 16 through the first gas flow path 60a. Oxygen diffuses to the blood side when passing through the lumen of the second hollow fiber membrane 16a. In addition, carbon dioxide in blood is discharged into the lumen of the second hollow fiber membrane 16a through the second hollow fiber membrane 16a. That is, gas exchange between oxygen and carbon dioxide is performed with blood through the second hollow fiber membrane 16a. Carbon dioxide in the lumen of the second hollow fiber membrane 16a is collected in the second gas flow path 60b and flows out to the outside through a gas outflow portion 78.

The blood introduced from the blood inflow portion 36 is guided to the blood flow path 28 (accommodation space S) through the blood introduction path 48. The blood in the blood flow path 28 passes through clearances of the heat exchanger 14 toward the outside in the radial direction in the accommodation space S. As a result, heat is exchanged between the blood and the heat medium in the lumen of the first hollow fiber membrane 14a.

The heat-exchanged blood flows radially outward in the accommodation space S through the intermediate spacer 18 and passes through the second hollow fiber membrane 16a of the gas exchanger 16. The blood performs gas exchange for absorbing oxygen that has passed through the second hollow fiber membrane 16a and releasing excessive carbon dioxide through the second hollow fiber membrane 16a. The blood subjected to the gas exchange flows in the blood flow path 28 in the circumferential direction, flows out of the oxygenator 10 through the blood outflow port 54, and returns to the human body.

Next, a method for manufacturing the oxygenator 10 will be described.

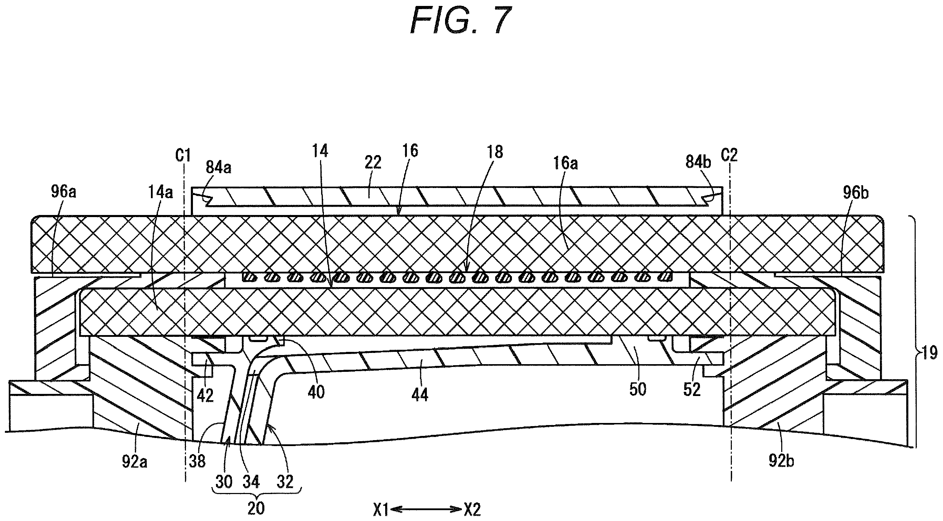

First, the hollow fiber module 19 is obtained that includes the heat exchanger 14, the intermediate spacer 18, and the gas exchanger 16 which are formed in order from the inner peripheral side as illustrated in Fig. 7. A first cap member 92a, a first annular member 96a, a second cap member 92b, and a second annular member 96b in Fig. 7 are members used only for manufacturing the hollow fiber module 19, and are removed in a removal step described later. Thereafter, the outer tube 22 is formed. The outer tube 22 is integrally formed by various resin molding methods such as injection molding.

Next, the hollow fiber module 19 and the outer tube 22 are assembled as illustrated in Fig. 7. Thus, the outer periphery of the gas exchanger 16 is covered by the outer tube 22. Thereafter, a cutting step is performed on the hollow fiber module 19. In the cutting step, the heat exchanger 14 and the gas exchanger 16 are cut along a first cutting line Cl and a second cutting line C2 in Fig. 7.

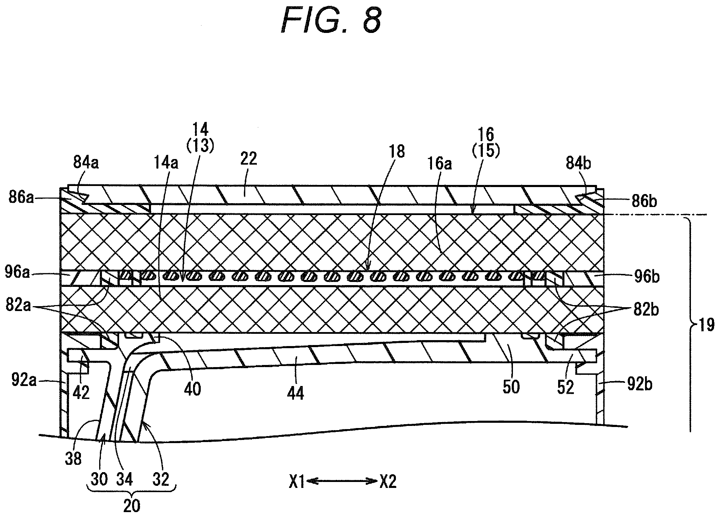

Next, a sealing step is performed as illustrated in Fig. 8. In the sealing step, the gap between the outer peripheral portion of the hollow fiber module 19 and the outer tube 22 is filled with the first sealing material 86a and the second sealing material 86b.

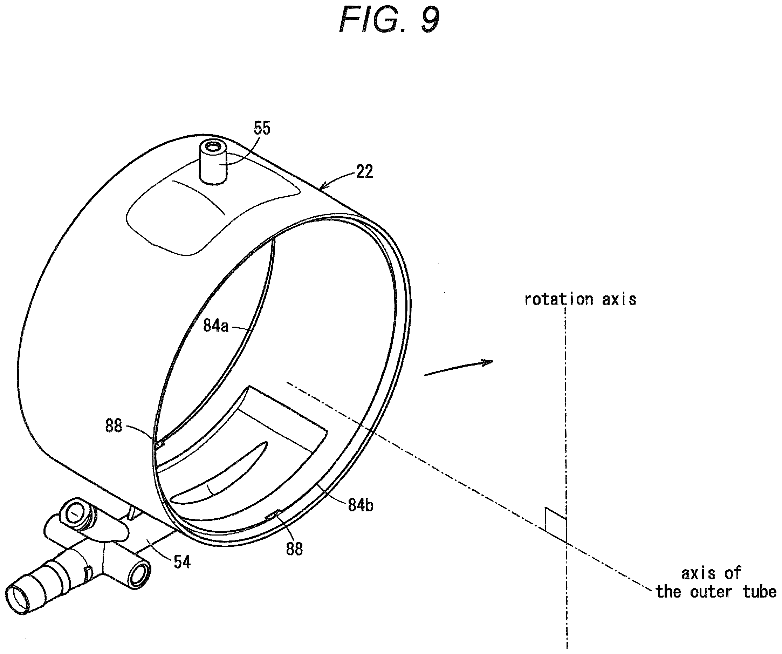

As illustrated in Fig. 9, the second sealing material 86b is disposed and fixed to a holding jig (not illustrated) such that the blood outflow port 54 of the outer tube 22 is located at the lower end and the prime auxiliary port 55 is located at the upper end. Therefore, the cutout portions 88 are disposed at the 0 o'clock position and the 6 o'clock position of the outer tube 22. The central axis of the outer tube 22 is oriented in a direction perpendicular to the rotation axis of the holding jig. Thereafter, a centrifugal force is applied in the axial direction (X1 direction) of the outer tube 22 by rotating the holding jig along the rotation axis.

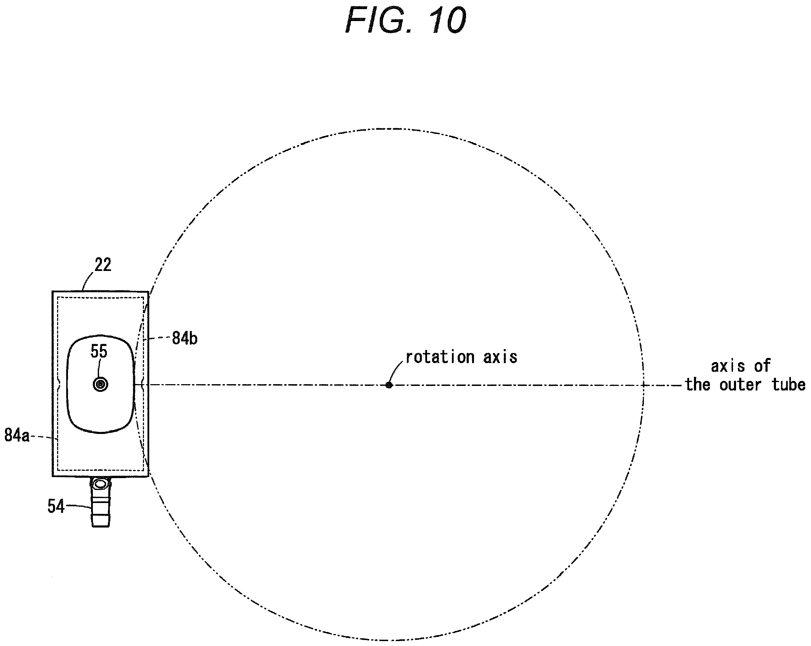

As illustrated in Fig. 10, the second anchor structure 84b formed at the other end of the outer tube 22 varies in distance from the rotation center depending on the position in the circumferential direction as viewed from above the rotation center. As illustrated, the 0 o'clock position and the 6 o'clock position of the outer tube 22 are closest to the rotation center. On the other hand, the 3 o'clock position and the 9 o'clock position of the outer tube 22 are the farthest from the rotation center. The centrifugal force is larger in a region farther from the rotation center. Therefore, in the second anchor structure 84b, the centrifugal force is the largest at the 3 o'clock position and the 9 o'clock position and is the smallest at the 0 o'clock position and the 6 o'clock position.

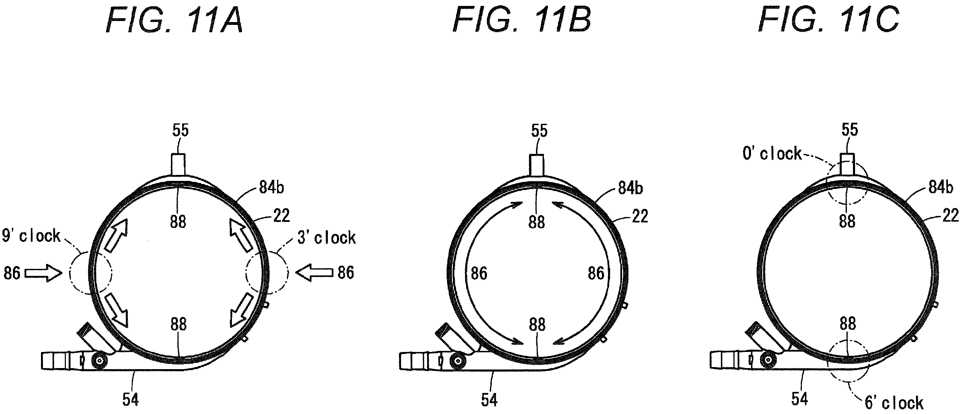

In the present embodiment, the second sealing material 86b (see Fig. 8) is introduced from the 3 o'clock position and the 9 o'clock position at which the centrifugal force is the largest as illustrated in Fig. 11A. As illustrated in Fig. 11B, the second anchor structure 84b is filled with the second sealing material 86b which spreads in the circumferential direction of the second anchor structure 84b under the action of the centrifugal force. Air in the second anchor structure 84b is discharged while being pushed out by the interface (interface with air) of the second sealing material 86b. The interface of the second sealing material 86b gradually advances from the 3 o'clock position and the 9 o'clock position toward the 0 o'clock position and the 6 o'clock position where the cutout portions 88 are provided. Accordingly, excess air is smoothly discharged from the second anchor structure 84b.

Finally, the interface of the second sealing material 86b reaches the vicinity of the cutout portions 88 having the smallest centrifugal force in the second anchor structure 84b, and thus, the cutout portions 88 are filled with the second sealing material 86b without leaving bubbles, as illustrated in Fig. 11C. Thereafter, the second sealing material 86b is cured, and thus, the filling with the second sealing material 86b is completed. The filling with the first sealing material 86a is also performed in the same manner as the second sealing material 86b.

Thereafter, in the removal step, the first cap member 92a, the first annular member 96a, the second cap member 92b, and the second annular member 96b are removed from the hollow fiber module 19. Thus, gaps are formed at one end and the other end of the heat exchanger 14 and the gas exchanger 16.

Then, in an attachment step, the first cover member 24a and the second cover member 24b are attached to one end and the other end of the outer tube 22 and the core 20, respectively, and are fixed with the adhesives 64a and 64b. During this process, the protruding end of the first partition wall 62a is inserted into a first gap, and the protruding end of a second partition wall 62b is inserted into a second gap. Thus, the oxygenator 10 is completed.

Examination Example 1 (Comparative Example) and Examination Example 2 (present embodiment) of the oxygenator 10 according to the present embodiment will be described below.

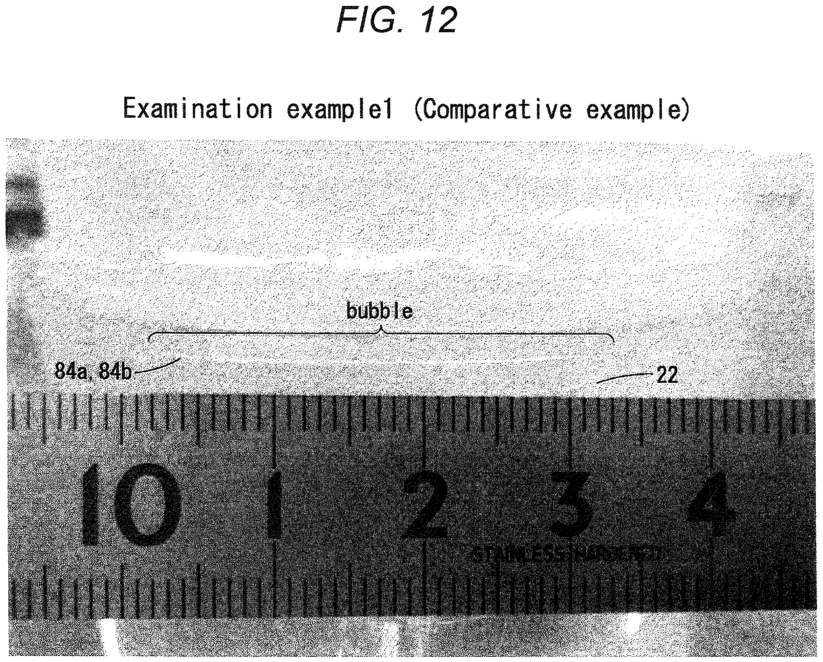

In Examination Example 1 (Comparative Example) illustrated in Fig. 12, the oxygenator 10 was filled with the second sealing material 86b without having the cutout portions 88 provided in the second anchor structure 84b. In Examination Example 1 (Comparative Example), bubbles remained in a state of being connected over a wide range in the circumferential direction of the second anchor structure 84b around the 0 o'clock position and the 6 o'clock position.

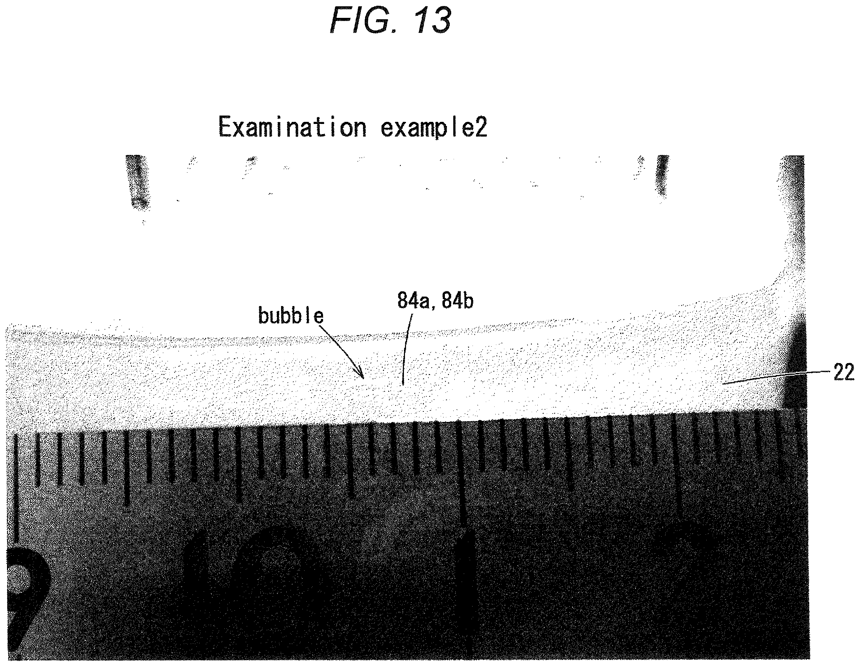

On the other hand, in Examination Example 2 (the present embodiment) illustrated in Fig. 13, a pair of cutout portions 88 was provided in the second anchor structure 84b, and filling with the second sealing material 86b was performed with the cutout portions 88 being disposed at the 0 o'clock position and the 6 o'clock position. In Examination Example 2, it was confirmed that most of bubbles in the second anchor structure 84b were discharged and bubbles could be prevented from remaining. As described above, it can be seen that, according to the present embodiment in which the cutout portions 88 are provided in the second anchor structure 84b, bubbles in the second sealing material 86b can be removed.

The oxygenator 10 according to the present embodiment has the following effects.

An oxygenator 10 according to the present embodiment includes: a hollow fiber module 19 having a plurality of hollow fiber membranes (for example, first hollow fiber membrane 14a and second hollow fiber membrane 16a); an outer tube 22 that is cylindrical and that accommodates the hollow fiber module 19; and a sealing structure (for example, first sealing structure 82a or second sealing structure 82b) that is provided at an end of the outer tube 22 and seals a gap between an outer peripheral portion of the hollow fiber module 19 and an inner peripheral portion of the outer tube 22, wherein the sealing structure includes an anchor structure (for example, first anchor structure 84a or second anchor structure 84b) that is formed on the outer tube 22 on an inner peripheral side near the end and that has a groove recessed in an axial direction of the outer tube 22, a cutout portion 88 formed by cutting out the groove of the anchor structure on the inner peripheral side, and a sealing material 86 (potting material) with which the anchor structure and the gap are filled.

With the above configuration, air in the anchor structure can be released through the cutout portion 88, which can reduce bubbles remaining in the sealing material 86.

In the oxygenator 10 described above, the cutout portion 88 may include a plurality of the cutout portions 88 which may be provided at positions facing each other in the circumferential direction of the anchor structure. With this configuration, when a centrifugal force is applied in the axial direction of the outer tube 22, air can be removed in a plurality of directions due to the pair of cutout portions 88 being disposed at positions where the centrifugal force is substantially equal.

In the oxygenator 10 described above, the groove of the anchor structure may be formed in the outer tube 22 on the inner peripheral side, and the cutout portion 88 may be formed by cutting out the groove on the inner peripheral side. With this configuration, the anchor structure is formed on the inner peripheral side of the outer tube 22, whereby separation of the sealing material 86 can be prevented, and bubbles remaining in the anchor structure can be reduced.

In the oxygenator 10 described above, the cutout portion 88 may be provided at a position where a centrifugal force is the smallest when the centrifugal force is applied in the axial direction of the outer tube 22. With this configuration, the cutout portion 88 is disposed at a last region of the anchor structure where the interface of the sealing material 86 reaches, whereby air pushed out by the interface of the sealing material 86 is smoothly discharged through the cutout portion 88.

The present embodiment provides a method for manufacturing an oxygenator 10 that includes a hollow fiber module 19 having a hollow fiber membrane that is wound, an outer tube 22 that is cylindrical and that accommodates the hollow fiber module 19, and a sealing structure that seals a gap between an outer peripheral portion of the hollow fiber module 19 and an inner peripheral portion of the outer tube 22, the sealing structure including an anchor structure that is formed at the end of the outer tube 22 and that has a groove formed over an entire circumference, a cutout portion 88 formed by cutting out a part of the groove of the anchor structure in a circumferential direction, and a sealing material 86 with which the anchor structure and the gap are filled, the method including: accommodating the hollow fiber module 19 into the outer tube 22; and filling the anchor structure and the gap between the outer tube 22 and the hollow fiber module 19 with the sealing material 86 while applying a centrifugal force in the axial direction of the outer tube 22.

According to the above method for manufacturing the oxygenator 10, air in the groove of the anchor structure is discharged through the cutout portion 88, whereby bubbles remaining in the sealing material 86 can be reduced.

In the method for manufacturing the oxygenator 10, during the filling, the sealing material 86 may be introduced with the cutout portion 88 being provided at a region where the centrifugal force is the smallest. With this method, the cutout portion 88 is disposed at the last region where the interface of the sealing material 86 reaches, whereby air pushed out by the interface of the sealing material 86 is smoothly discharged through the cutout portion 88.

In the method for manufacturing the oxygenator 10, during the filling, the sealing material 86 may be introduced from a region where the centrifugal force acting on the sealing material 86 is the largest. According to this method, the interface of the sealing material 86 advances from the region where the centrifugal force is the largest toward the region where the centrifugal force is the smallest, whereby air in the anchor structure can be smoothly discharged from the cutout portion 88.

### (Second Embodiment)

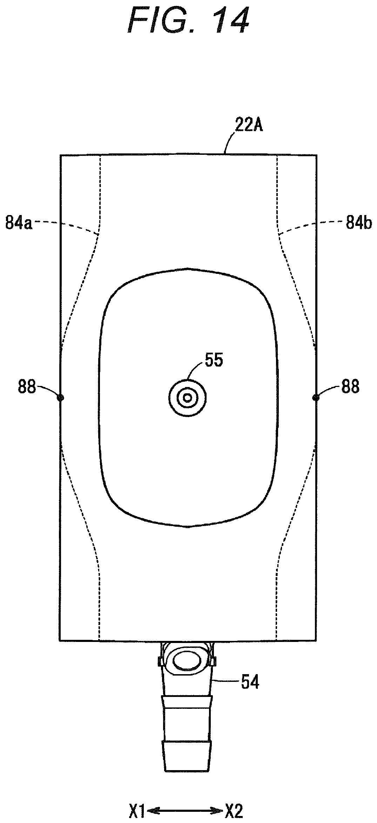

As illustrated in Fig. 14, an outer tube 22A of the present embodiment is provided with a first anchor structure 84a and a second anchor structure 84b which meander in the axial direction. The first anchor structure 84a and the second anchor structure 84b meander so as to be closest to the end of the outer tube 22A at the 0 o'clock position and the 6 o'clock position and farthest from the end of the outer tube 22A at the 3 o'clock position and the 9 o'clock position. In the first anchor structure 84a and the second anchor structure 84b, cutout portions 88 are formed at positions closest to the end of the outer tube 22A.

The first anchor structure 84a and the second anchor structure 84b meander such that the regions where the cutout portions 88 are formed are located closest to the rotation center when the centrifugal force is applied, regardless of how the outer tube 22A is disposed. Therefore, during the process of introducing the first sealing material 86a or the second sealing material 86b, the cutout portions 88 are always disposed in the region having the smallest centrifugal force regardless of the orientation of the outer tube 22A in the circumferential direction. Accordingly, the same effects as those of the outer tube 22 of the first embodiment (see Fig. 3 and the like) can be obtained by the outer tube 22A of the present embodiment.

While the present invention has been described above with reference to preferred embodiments, it is obvious that the present invention is not limited to the above embodiments, and that various modifications are possible without departing from the gist of the present invention.

## Claims

1. An oxygenator comprising:
a hollow fiber module having a plurality of hollow fiber membranes;
an outer tube that is cylindrical and that accommodates the hollow fiber module; and
a sealing structure that is provided at an end of the outer tube and seals a gap between an outer peripheral portion of the hollow fiber module and an inner peripheral portion of the outer tube, wherein
the sealing structure includes
an anchor structure that is formed on the outer tube on an inner peripheral side near the end and that has a groove recessed in an axial direction of the outer tube,
a cutout portion formed by cutting out the groove of the anchor structure on the inner peripheral side, and
a sealing material with which the anchor structure and the gap are filled.

2. The oxygenator according to claim 1, wherein the cutout portion includes a plurality of the cutout portions that is provided at regions of the anchor structure facing each other in a circumferential direction.

3. The oxygenator according to claim 1 or 2, wherein the groove of the anchor structure is formed in the outer tube on the inner peripheral side, and the cutout portion is formed by cutting out the groove on the inner peripheral side.

4. The oxygenator according to any one of claims 1 to 3, wherein the cutout portion is provided at a position where a centrifugal force is the smallest when the centrifugal force is applied in the axial direction of the outer tube.

5. A method for manufacturing an oxygenator that includes a hollow fiber module having a plurality of hollow fiber membranes, an outer tube that is cylindrical and that accommodates the hollow fiber module, and a sealing structure that is provided at an end of the outer tube and that seals a gap between an outer peripheral portion of the hollow fiber module and an inner peripheral portion of the outer tube, the sealing structure including an anchor structure that is formed on the outer tube on an inner peripheral side near the end and that has a groove recessed in an axial direction of the outer tube, a cutout portion formed by cutting out the groove of the anchor structure on the inner peripheral side, and a sealing material with which the anchor structure and the gap are filled, the method comprising:
accommodating the hollow fiber module into the outer tube; and
filling the groove and the gap between the outer tube and the hollow fiber module with the sealing material while applying a centrifugal force in the axial direction of the outer tube.

6. The method for manufacturing an oxygenator according to claim 5, wherein, during the filling, the sealing material is introduced with the cutout portion being provided at a region where the centrifugal force is the smallest.

7. The method for manufacturing an oxygenator according to claim 5 or 6, wherein, during the filling, the sealing material is introduced from a region where the centrifugal force acting on the sealing material is the largest.
